(19) **Europäisches Patentamt / European Patent Office / Office européen des brevets**

(11) **EP 3 010 552 B1**

(12) **EUROPEAN PATENT SPECIFICATION**

(45) Date of publication and mention of the grant of the patent:
**17.04.2019 Bulletin 2019/16**

(21) Application number: **14731952.9**

(22) Date of filing: **19.06.2014**

(51) Int Cl.:
*A61K 49/04* [(2006.01)]    *A61P 35/00* [(2006.01)]

(86) International application number:
**PCT/EP2014/062976**

(87) International publication number:
**WO 2014/202738 (24.12.2014 Gazette 2014/52)**

(54) **COMPOSITIONS FOR USE IN ONCOLOGY**

ZUSAMMENSETZUNGEN ZUR VERWENDUNG IN DER ONKOLOGIE

COMPOSITIONS POUR UNE UTILISATION EN ONCOLOGIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.06.2013 EP 13305830**
       **20.06.2013 US 201361837406 P**

(43) Date of publication of application:
**27.04.2016 Bulletin 2016/17**

(73) Proprietor: **Nanobiotix**
**75012 Paris (FR)**

(72) Inventors:
• **POUL, Laurence**
  **75012 Paris (FR)**
• **LEVY, Laurent**
  **75014 Paris (FR)**
• **BERJAUD, Céline**
  **94700 Maisons-Alfort (FR)**
• **GERMAIN, Matthieu**
  **77163 Dammartin sur Tigeaux (FR)**
• **POTTIER, Agnès**
  **75006 Paris (FR)**

(74) Representative: **Cabinet Becker et Associés**
**25, rue Louis le Grand**
**75002 Paris (FR)**

(56) References cited:
**WO-A2-2011/084465    US-A- 5 370 901**

• **ZHI-XIAO GUO ET AL: "Generation of alginate gel particles with AuNPs layers by polydimethylsiloxan template", BIOMICROFLUIDICS, vol. 5, no. 2, 1 January 2011 (2011-01-01) , page 026502, XP055083546, ISSN: 1932-1058, DOI: 10.1063/1.3602119**
• **SACHIEIKEGAMI ET AL: "THE JOURNAL OF NUTRITION", CARBOHYDRATE AND FIBER EFFECT OF VISCOUS INDIGESTIBLE POLYSACCHARIDES ON PANCREATIC-BILIARY SECRETION AND DIGESTIVE ORGANS IN RATS, vol. 120, 1 January 1990 (1990-01-01), pages 353-360, XP055083570,**
• **MANSOR BIN AHMAD ET AL: "Synthesis of Silver Nanoparticles in Chitosan, Gelatin and Chitosan/Gelatin Bionanocomposites by a Chemical Reducing Agent and Their Characterization", MOLECULES, vol. 16, no. 12, 25 August 2011 (2011-08-25), pages 7237-7248, XP055083626, DOI: 10.3390/molecules16097237**
• **LAURENCE MAGGIORELLA ET AL: "Nanoscale radiotherapy with hafnium oxide nanoparticles", FUTURE ONCOLOGY, FUTURE MEDICINE LTD., LONDON, GB , vol. 8, no. 9 1 September 2012 (2012-09-01), pages 1167-1181, XP002698557, ISSN: 1479-6694, DOI: 10.2217/FON.12.96 Retrieved from the Internet: URL:http://www.futuremedicine.com/doi/abs/ 10.2217/fon.12.96**

**(Cont. next page)**

• WITTEVEEN JOLANDA ET AL: "Gelatin/glycerol coating to preserve mechanically compliant nanowire electrodes from damage during brain implantation", JOURNAL OF VACUUM SCIENCE AND TECHNOLOGY: PART B, AVS / AIP, MELVILLE, NEW YORK, NY, US, vol. 28, no. 6, 11 November 2010 (2010-11-11), pages C6K13-C6K16, XP012144368, ISSN: 1071-1023, DOI: 10.1116/1.3498764

Remarks:
The file contains technical information submitted after the application was filed and not included in this specification

**Description**

## FIELD OF THE INVENTION

The disclosure relates to compositions and methods for use in medical diagnostic and patient monitoring, typically in the context of therapy, in particular in the context of oncology to optimize tumor bed local irradiation. It more particularly relates to a biocompatible gel comprising nanoparticles and/or nanoparticles aggregates, wherein i) the density of each nanoparticle and of each nanoparticles aggregate is of at least 7 g/cm$^3$, the nanoparticle or nanoparticles aggregate comprising an inorganic material comprising at least one metal element having an atomic number Z of at least 25, more preferably of at least 40, each of said nanoparticle and of said nanoparticles aggregate being covered with a biocompatible coating; ii) the nanoparticles and/or nanoparticles aggregates concentration is of at least about 1% (w/w); and iii) the apparent viscosity at 2 s$^{-1}$ of the gel comprising nanoparticles and/or nanoparticles aggregates, at is between about 0.1 Pa.s and about 1000 Pa.s when measured between 20°C and 37°C.

The composition of the invention typically allows improving the post-surgery tumor bed delineation in order to optimize its irradiation.

## BACKGROUND

The local control of cancer disease's recurrence or relapse constitutes a crucial step of anticancer treatment following surgery and radiotherapy steps. Post-operative radiotherapy is used in several indications to treat the tumor bed once tumorectomy has been performed in order to improve rates of local control and thus reduce, ideally avoid, tumor recurrences. A recent meta-analysis of the Early Breast Cancer Trialists' Collaborative Group stressed the importance of reducing local breast tumor recurrences, because one breast cancer death could be avoided for every four local recurrences avoided. According to the authors of "Customized computed tomography-based boost volumes in breast-conserving therapy: use of three-dimensional histologic information for clinical target volume margins" [IJROBP 75(3): 757-763 (2009)], one method to improve local control is to increase the irradiation dose the tumor bed is exposed to (i.e. boost irradiation). The authors add that this effect could be further increased by improving the delineation of the tumor bed (i.e., the target volume the boost irradiation should specifically target).

The International Commission on Radiation Units and Measurements defines the Gross Tumor Volume (GTV) as the gross demonstrable extent and location of a malignant growth. For the adjuvant breast radiotherapy (the surgical step is followed by a radiotherapy step), the GTV has been excised with a variable margin of tissue, leaving a cavity. The cavity is not the GTV, but related to it. The cavity walls is referred to, somewhat loosely, as the tumor bed ["Target volume definition for external beam partial breast radiotherapy: clinical, pathological and technical studies informing current approaches" Radiotherapy and Oncology 94 255-263 (2010)].

In clinical practice, accurately identifying the tumor bed is challenging and a high rate of inter-observer variability in tumor bed contouring is frequently reported, especially in poorly visualized resection cavities ["Excised and Irradiated Volumes in Relation to the Tumor size in Breast-Conserving Therapy" Breast Cancer Res Treat 129:857-865 (2011)]. The irradiated postoperative volume (as delineated on the radiotherapy planning CT-scan before the start of radiotherapy), in patients treated with breast-conserving therapy is not, for most of the cases, clearly visible and a cavity visualization score is frequently used to assess the quality of the irradiated postoperative volume identification.

Likewise, for prostate cancers, the EORTC Radiation Oncology Group has made recommendation for target volume definition in post-operative radiotherapy, presenting guidelines for standardization of the target volume definition and delineation as well as standardization of the clinical quality assurance procedures; authors from "Guidelines for target volume definition in post-operative radiotherapy for prostate cancer, on behalf of the EORTC Radiation Oncology Group" [Radiotherapy & Oncology 84 121-127 (2007)], in particular referred to a study where a high inter-observer variability of target volume delineation in postoperative radiotherapy for prostate cancer was observed when performed by five (5) distinct radiation oncologists for eight (8) distinct patients (The CTV varied between the physicians from 39 to 53 cm$^3$ for the patient corresponding to the smallest variation and from 16 to 69 cm$^3$ for the patient corresponding to the largest variation).

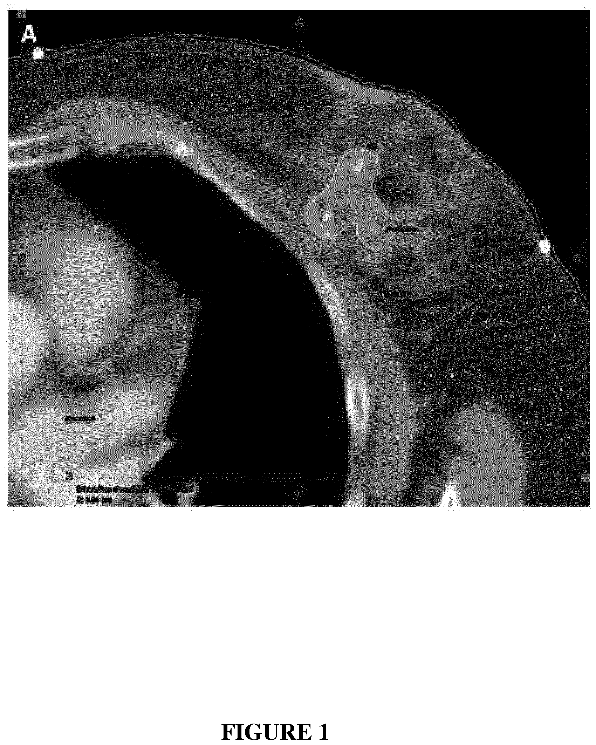

A study to evaluate the accuracy of a boost technique, reported in "Improving the definition of the tumor bed boost with the use of surgical clips and image registration in breast cancer patients" [Int. J. Radiation Oncology Biol. Phys. Vol 78(5) ; 1352-1355 (2010)], shows that the use of radiopaque clips during tumorectomy, typically the use of 3 or more clips, increases the accuracy of the tumor bed delineation (cf. Figure 1). However, questions over the accuracy of CT/clip-based TB delineation remain. Clips only define points located on the excision cavity walls such that the remaining tumor tissue-excision cavity interface must be derived by interpolation, taking into account tissue density and distortion.

Interestingly, a report on the magnitude of volumetric change in the postlumpectomy tumor bed has demonstrated significant tumor bed volume changes before and during radiation therapy or radiotherapy (RT) ["The dynamic tumor bed: volumetric changes in the lumpectomy cavity during breast conserving therapy" Int. J. Radiation Oncology Biol. Phys. 74(3):695-701 (2009)]. Thirty-six (36) patients were enrolled in the study, with Tis (10), T1 (24) and T2 (2) breast tumors. Thirty (30) patients received a whole breast irradiation after lumpectomy followed by a boost dose of 10 Gy. Six (6) patients were treated with partial breast irradiation. Treatment planning CT scans of the breast were obtained shortly after surgery, before the start of the whole breast irradiation for treatment planning and before delivery of the tumor bed boost. Patients who were treated with partial breast irradiation received only a scan postoperatively and a scan before tumor bed treatment.

During the interval between postoperative scan and second scan (median interval, 3 weeks), the tumor bed volume decreased by a median of 49.9%. Between the planning scan and the boost scan (median interval, 7 weeks), the median tumor bed volume decreased by 44.6%.

A subgroup of eight (8) patients, who experienced a delay (median interval, 23 weeks) between surgery and RT because of planned chemotherapy, had a median reduction of the tumor bed volume of 60.3% during the interval between postoperative scan and planning scan. When this magnitude and rate-of-change data were evaluated in context of the entire patient set, the observed results suggested that the tumor bed volume decreased more rapidly in the weeks immediately after surgery and then attained a relative plateau. According to the authors, the impact of large volumetric change on planning volume, dosimetry, or clinical parameters such as local control or cosmetic outcome, is an important area for future research as theoretically, if a single planning scan is used to plan the boost clinical target volume (CTV) in a patient with a tumor bed that shrinks dramatically during the course of RT, the surrounding normal tissues receive unnecessary additional radiation that could yield poorer cosmetic outcomes and more late undesirable effects. Conversely, if a single planning scan is performed long after surgery, the reduced tumor bed volume could actually result in underestimating the true tumor bed or the area of surgical tumor contamination.

WO 2011/084465 relates to stabilizing and visualizing tissues gaps left by surgical removal of cancerous tissues. According to the inventors, a conformal filling approach is a considerable improvement over the use of clips, which provide poor resolutions of the site's margin. The described implants may be formulated to be stable until no longer needed, and then biodegrade. According to WO 2011/084465, the implantation of the hydrogel, leads to an increase of the mean cavity volume. Therefore, when using standard margins, the hydrogel tends to increase normal tissue radiation doses. A reduced margin expansion is thus required in order to decrease normal tissue radiation doses.

As easily understandable from the above, there remains a clear need to improve the post-surgery tumor bed delineation in order to optimize irradiation to the tumor bed only.

## DETAILED DESCRIPTION

Inventors now provide an advantageous composition which considerably improves targeted tissue delineation, in particular tumor bed delineation, without impacting on targeted tissue volume changes, typically when considering a tumor bed, on tumor bed volumes changes or on post lumpectomy tissue remodeling. In the context of the invention, the tumor bed is tissue covering the cavity obtained following tumor resection.

The composition of the invention further advantageously allows an at least 10% increase of energy (radiation) dose deposit on the tumor bed, i.e. without increasing the energy dose deposition in surrounding healthy tissues.

A first object herein disclosed relates to a biocompatible gel comprising nanoparticles and/or nanoparticles aggregates, wherein i) the density of each nanoparticle and of each nanoparticles aggregate is of at least 7 g/cm$^3$, the nanoparticle or nanoparticles aggregate comprising an inorganic material comprising at least one metal element having an atomic number Z of at least 25, more preferably of at least 40, each of said nanoparticle and of said nanoparticles aggregate being covered with a biocompatible coating; ii) the nanoparticles and/or nanoparticles aggregates concentration is of at least about 1% (w/w); and iii) the apparent viscosity at 2s$^{-1}$ of the gel comprising nanoparticles and/or nanoparticles aggregates, is between about 0.1 Pa.s and about 1000 Pa.s when measured between 20°C and 37°C.

Nanoparticles and/or nanoparticles aggregates are typically embedded in the gel of the invention.

The biocompatible gel comprising nanoparticles and/or nanoparticles aggregates according to the invention advantageously allows the delineation and visualization of at least 40%, preferably at least 50%, even more preferably more than 50%, of a target biological tissue when the target biological tissue is observed using X-ray imaging equipment.

The targeted biological tissue is typically a tumor bed.

In a preferred embodiment, when the gel is applied on a target biological tissue, nanoparticles and/or nanoparticles aggregates of the biocompatible gel allow a at least about 10% increase of the radiation dose deposit on said target biological tissue when exposed to ionizing radiations, when compared to the dose deposit on the same biological tissue in the absence of said gel.

## INORGANIC NANOPARTICLE

In the present description, the terms "nanoparticle(s)", "nanoparticles aggregate(s)" and "particle(s)" are indifferently used.

In the context of the present invention, the terms "nanoparticle" or "nanoparticles aggregate" refer to a product, in particular a synthetic product, with a size in the nanometer range, typically between 1 nm and 500 nm.

The size of the nanoparticle and its structure and composition may be analyzed from X-ray diffractogram.

The term "aggregate of nanoparticles" or "nanoparticles aggregate" refers to an assemblage of nanoparticles strongly, typically covalently, bound to each other.

The terms "size of the nanoparticle" or "size of the nanoparticles aggregate" and "largest size of the nanoparticle" or "largest size of the nanoparticles aggregate" herein refer to the "largest dimension of the nanoparticle" or "largest dimension of the nanoparticles aggregate" or "diameter of the nanoparticle" or "diameter of the nanoparticles aggregate". Transmission Electron Microscopy (TEM) can be used to measure the size of the nanoparticle or nanoparticles aggregate. As well, Dynamic Light Scattering (DLS) can be used to measure the hydrodynamic diameter of nanoparticles or nanoparticles aggregates in solution. These two methods may further be used one after each other to compare size measures and confirm said size.

The largest dimension of a nanoparticle or nanoparticles aggregate as herein defined is typically between about 5 nm and about 250 nm, preferably between about 10 nm and about 100 nm or about 200 nm, even more preferably between about 20 nm and about 150 nm.

As the shape of the particle can influence its "biocompatibility", particle having a quite homogeneous shape is preferred. For pharmacokinetic reasons, nanoparticles or nanoparticles aggregates being essentially spherical, round or ovoid in shape are thus preferred. Such a shape also favors the nanoparticle or nanoparticles aggregate interaction with or uptake by cells. Spherical or round shape is particularly preferred.

Typically, the largest dimension is the diameter of a nanoparticle or nanoparticles aggregate of round or spherical shape, or the longest length of a nanoparticle or nanoparticles aggregate of ovoid or oval shape.

According to the disclosure, the inorganic material the nanoparticle or nanoparticles aggregate is prepared with, typically comprises at least one metal element, typically a metal element having an atomic number Z of at least 25, preferably of at least 40, even more preferably above 40. The inorganic material can also comprise several metal elements, typically two metal elements.

In the claimed invention, the nanoparticle or nanoparticles aggregate consists in an inorganic material, said inorganic material comprising a single metal element or a mixture of metal elements.

According to the disclosure, the inorganic material is preferably a material having an effective atomic number ($Z_{eff}$) of at least 25, preferably at least 40 or 41, more preferably at least 50 or 51, more preferably at least 60, 61, 62 or even 63.

Effective atomic number is a term that is similar to atomic number but is used for compounds (e.g. water) and mixtures of different materials (such as tissue and bone) rather than for atoms. Effective atomic number calculates the average atomic number for a compound or mixture of materials. It is abbreviated $Z_{eff}$.

The effective atomic number is calculated by taking the fractional proportion of each atom in the compound and multiplying that by the atomic number of the atom. The formula for the effective atomic number, $Z_{eff}$, is as follows:

$$Z_{eff} = \sqrt[2.94]{f_1 \times (Z_1)^{2.94} + f_2 \times (Z_2)^{2.94} + f_3 \times (Z_3)^{2.94} + \ldots}$$

where

$f_n$ is the fraction of the total number of electrons associated with each element, and
$Z_n$ is the atomic number of each element.

The atomic number (also known as the proton number) is the number of protons found in the nucleus of an atom. It is traditionally represented by the symbol Z (and is herein also identified as $Z_n$). The atomic number uniquely identifies a chemical element. In an atom of neutral charge, atomic number is equal to the number of electrons.

An example is that of water ($H_2O$) which is made up of two hydrogen atoms (Z=1) and one oxygen atom (Z=8). The total number of electrons is 1+1+8 = 10. The fraction of electrons corresponding to the two hydrogens is 2/10 and the fraction of electrons corresponding to the unique oxygen is (8/10). $Z_{eff}$ of water is therefore:

$$Z_{eff} = \sqrt[2.94]{0.2 \times 1^{2.94} + 0.8 \times 8^{2.94}} = 7.42$$

$Z_{eff}$ participate to the incoming radiations absorption capacity of nanoparticles.

The inorganic material constituting the nanoparticle and/or nanoparticles aggregate is typically selected from a metal, an oxide, a sulfide and any mixture thereof. As herein disclosed, this inorganic material comprises at least one metal element having an atomic number Z of at least 25, preferably of at least 40, even more preferably above 40.

The nanoparticle or nanoparticle aggregate used in the gel of the invention consists of an inorganic material, wherein the density of said nanoparticle and nanoparticle aggregate is of at least 7 g/cm$^3$.

When the inorganic material constituting the nanoparticle and/or nanoparticles aggregate is an oxide, this oxide may be selected for example from cerium (IV) oxide ($CeO_2$), neodynium (III) oxide ($Nd_2O_3$), samarium (III) oxide ($Sm_2O_3$), europium (III) oxide ($Eu_2O_3$), gadolinium (III) oxide ($Gd_2O_3$), terbium (III) oxide ($Tb_2O_3$), dysprosium (III) oxide ($Dy_2O_3$), holmium oxide ($Ho_2O_3$), erbium oxide ($Er_2O_3$), thullium (III) oxide ($Tm_2O_3$), ytterbium oxide ($Yb_2O_3$), lutetium oxide ($Iu_2O_3$), hafnium (IV) oxide ($HfO_2$), tantalum (V) oxide ($Ta_2O_5$), rhenium (IV) oxide ($ReO_2$), bismuth (III) oxide ($Bi_2O_3$). In a particular embodiment, a mixture of oxides can also be used as the inorganic material to prepare the nanoparticle and/or nanoparticles aggregate of the invention. Nanoparticle and/or nanoparticles aggregate of the invention can thus comprise or consist in a mixture of oxides.

When the inorganic material constituting the nanoparticle and/or nanoparticles aggregate is a metal, this metal may be selected for example from gold metal (Au), silver metal (Ag), platinum metal (Pt), palladium metal (Pd), tin metal (Sn), tantalum metal (Ta), hafnium metal (Hf), terbium metal (Tb), thulium metal (Tm), dysprosium metal (Dy), erbium metal (Er), holmium metal (Ho), iron metal (Fe), neodymium metal (Nd), and lutetium metal (Lu). As indicated previously, in a particular embodiment, a mixture of metals can also be used as the inorganic material to prepare the nanoparticle and/or nanoparticles aggregate of the invention. Nanoparticle and/or nanoparticles aggregate of the invention can thus comprise or consist in a mixture of metals.

When the inorganic material constituting the nanoparticle and/or nanoparticles aggregate is a sulfide, this sulfide is preferably silver sulfide ($Ag_2S$).

A mixture of an oxide, of a metal and/or of a sulfide can also be used to prepare the nanoparticles and/or nanoparticles aggregates. Herein described nanoparticle and/or nanoparticles aggregate can thus comprise or consist in a mixture of an oxide, a metal and/or a sulphide.

An example of nanoparticle which can advantageously be used in the context of the present invention is a gold metal nanoparticle covered with hafnium oxide material.

In a preferred embodiment, the nanoparticle and/or nanoparticles aggregate used in the context of the present invention can be coated with a biocompatible material selected from an agent displaying a steric group. Such a group may be selected for example from polyethylene glycol (PEG); polyethylenoxide; polyvinylalcohol; polyacrylate; polyacrylamide (poly(N-isopropylacrylamide)); polycarbamide; a biopolymer; a polysaccharide such as dextran, xylan and cellulose; collagen; and a switterionic compound such as polysulfobetain; etc.

In another preferred embodiment, the nanoparticle and/or nanoparticles aggregate used in the context of the invention can be coated with a biocompatible material selected from an agent allowing interaction with a biological target. Such an agent can typically bring a positive or a negative charge on the nanoparticle's surface. This charge can be determined by zeta potential measurements, typically performed on nanoparticles and/or nanoparticles aggregates suspensions the concentration of which vary between 0.2 and 10 g/L, the nanoparticles and/or nanoparticles aggregates being suspended in an aqueous medium with a pH comprised between 6 and 8.

An agent forming a positive charge on the nanoparticle surface can be for example aminopropyltriethoxysilane or polylysine. An agent forming a negative charge on the nanoparticle surface can be for example a phosphate (for example a polyphosphate, a metaphosphate, a pyrophosphate, etc.), a carboxylate (for example citrate or dicarboxylic acid, in particular succinic acid) or a sulphate.

Advantageously, the coating preserves the integrity of the nanoparticle and/or nanoparticles aggregate *in vivo,* ensures or improves the biocompatibility thereof, and facilitates an optional functionalization thereof (for example with spacer molecules, biocompatible polymers, targeting agents, proteins, etc.). In addition, the coating is advantageously used in the context of the present invention to facilitate the binding of the particle with the targeted biological tissue or cell.

A particular nanoparticle and/or nanoparticles aggregate as herein disclosed can further comprise at least one targeting agent allowing its interaction with a recognition element present on the target cell. Such a targeting agent typically acts once the nanoparticles and/or nanoparticles aggregates delineate the target site. The targeting agent can be any biological or chemical structure displaying affinity for molecules present in the human or animal body. For instance it can be a peptide, oligopeptide or polypeptide, a protein, a nucleic acid (DNA, RNA, SiRNA, tRNA, miRNA, etc.), a hormone, a vitamin, an enzyme, the ligand of a molecule expressed by a pathological cell, in particular the ligand of a tumor antigen, hormone receptor, cytokine receptor or growth factor receptor. Said targeting agents can be selected for example in the group consisting in LHRH, EGF, a folate, anti-B-FN antibody, E-selectin/P-selectin, anti-IL-2R$\alpha$ antibody, GHRH, etc.

## BIOCOMPATIBLE GEL

A natural polymer gel is mainly obtained by the formation of intermolecular bounds as a result i) of temperature and pH changes and ii) of the presence of metallic ions. Thus, during the formation of the gel, a reversible solution-gel transition takes place.

On the other hand, synthetic gels consist of polymer chains connected by covalent bonds or other physical bonds. These structures typically lead to irreversible gel formation.

The properties of gels are influenced by both networks and solvents. A gel swells when immersed in a good solvent. Hydrogels are gels that typically swell in aqueous environments.

A preferred biocompatible gel according to the invention is a biocompatible hydrogel.

Polymers used for medical applications are to be biocompatible; i.e. upon contact with a body, for example with internal organs or with any other biological systems, they should not cause inflammation and/or adverse reactions.

Typical polymers which can be used to form the biocompatible gel can be selected from polyethyleneimine (PEI); polyethyleneglycol (PEG); polypropyleneglycol (PPG); polysaccharide, including for example cellulose derivatives (for example methylcellulose, carboxymethylcellulose, hydroxyethylcellulose, hydroxypropylcellulose), hyaluronic acid derivatives, chitosan, dextran, etc; poly(acrylamide) derivatives; poly(lactic acid) (PLA) derivatives; poly(acrylic acid) (PAA) derivatives; Poly (lactide-co-glycolic) acid (PLGA) derivatives; Polyvinyl alcohol (PVA); Poly(vinylpyrrolidone); Polyalkylcyanoacrylate derivatives; collagen derivatives; poly(glutamic acid) (PGA); and gelatin. The biocompatible gel can also be composed of any mixture of the herein identified polymers. Preferred polymers which can be advantageously used to prepare biocompatible hydrogels can be selected in the polysaccharide family which includes i) cellulose derivatives, typically methylcellulose, carboxymethylcellulose, hydroxyethylcellulose, hydroxypropylcellulose, and ii) members of the hyaluronic acid family or derivatives thereof typically obtained by introducing a functional group onto the hyaluronic acid. This can be achieved by formation of an active ester at the carboxylate of the glucuronic acid moiety of the hyaluronic acid and by subsequent substitution thereof with an amino or aldehyde group.

In order to modulate the viscosity of the gel, these polysaccharides can be cross-linked typically with low- or high-molecular-weight cross linkers or can be auto-cross-linked (using physical means such as heat or radiations and in the absence of any foreign molecules).

The quantity of polymer to be dispersed in a solvent in order to form a biocompatible gel according to the invention is typically between 0.1% and 50% (weight by weight w/w), more preferably between 0.5% and 40%, typically between 0.5% and 35%, or between 0.5% and 25%, and even more preferably between about 1%, about 2%, or about 3% and about 15% or about 20% (w/w).

When the biocompatible gel is a hydrogel, the solvent is typically an aqueous medium.

The apparent viscosity at $2s^{-1}$ of the biocompatible gel comprising nanoparticles and/or nanoparticles aggregates, for a temperature between 20°C and 37°C, is between about 0.1 Pa.s and about 1000 Pa.s, preferably between 1 Pa.s and 750 Pa.s, typically between 5 Pa.s and 500 Pa.s or 5 Pa.s and 300 Pa.s. Viscosity measurement is typically performed, at 20°C and 37°C, using a Couette rheometer (MODEL RM200, LAMY Rheology) on a given range of shear rate, lying between $0.1 \, s^{-1}$ and $300 \, s^{-1}$. The apparent viscosity is reported at $2s^{-1}$.

For each sample, the measurement is carried out on a volume of at least 25 ml with the suitable spindle, following the standard DIN ISO 3219 recommendations.

## INTERACTION PARTICLE-GEL

In the biocompatible gel comprising nanoparticles and/or nanoparticles aggregates herein disclosed, each nanoparticle or nanoparticle aggregate comprise or consist in an inorganic material, typically in an inorganic material comprising at least one metal element having an atomic number Z of at least 25, preferably of at least 40, and the density of each nanoparticle and nanoparticles aggregate is of at least $7 \, g/cm^3$. Each nanoparticle or nanoparticle aggregate is advantageously covered with a biocompatible coating.

The nanoparticles and/or nanoparticles aggregates concentration within the gel is of at least about 1% (w/w). In a preferred embodiment, the nanoparticles and/or nanoparticles aggregates concentration within the gel is between about 1.5% and 50% (w/w), preferably between 1.5% and 25% (w/w), even more preferably between 1.5% and 10% (w/w) or 1.5% and 5% (w/w), typically between 2% and 4% (w/w). For example, the nanoparticles and/or nanoparticles aggregates concentration within the gel is equal to about 1.5%, 2%, 3.5%, 4% or 5% (w/w).

The absence of any strong interaction (a strong interaction being typically a covalent interaction) between the nanoparticles and/or nanoparticles aggregates and the polymer which forms the biocompatible gel is an important feature to ensure that said nanoparticles and/or nanoparticles aggregates are actually released from the gel in order for them to correctly delineate the tumor bed.

The absence of strong interaction between the particle and the polymer which forms the biocompatible gel can typically be verified by measuring the viscosity of the gel comprising the nanoparticles and/or nanoparticles aggregates at 20°C and 37°C, as described above, and by comparing the obtained viscosity curve with that of a gel comprising neither nanoparticles nor nanoparticles aggregates. Similar viscosity curves (i.e. values differing one from each other by no more than 20%, typically by no more than 15%) confirm the absence of strong interaction between nanoparticles and/or nanoparticles aggregates and gel.

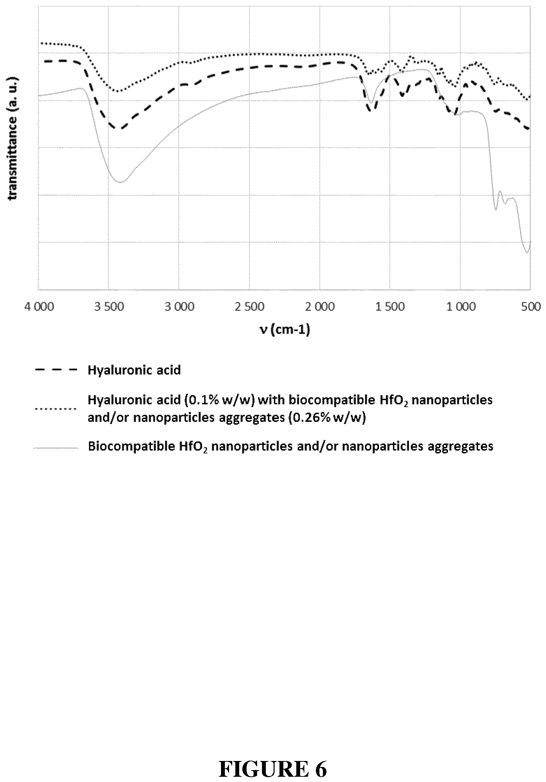

The absence of strong interaction between the particle and the polymer which forms the biocompatible gel can also be typically verified by Fourier Transformation Infra-Red (FTIR), by measuring the transmittance spectrum (in function of the wavenumber) of the gel comprising the nanoparticles and/or nanoparticles aggregates, and by comparing the obtained spectrum with that of a gel comprising neither nanoparticles nor nanoparticles aggregates and also with that of nanoparticles or nanoparticles aggregates. Transmittance spectrum of the gel comprising nanoparticles and/or nanoparticles aggregates strictly corresponds to the addition of the transmittance spectrum of gel plus the transmittance spectrum of nanoparticles or nanoparticles aggregates. This transmittance spectrum does not reveal any further band. This confirms the absence of strong interactions between the gel according to the invention and nanoparticles or nanoparticles aggregates (cf. examples 10 and 11 and Figure 6).

## BIOLOGICAL TISSUES AND TUMOR BED DELINEATION AND VISUALIZATION

Classically used methods for tumor bed visualization and treatment planning (i.e. planning of the appropriate radiotherapy) include clinical methods such as i) planning using the palpation and/or the surgical scar; ii) planning taking into account pre-surgical imaging findings (typically mammography), clinical history and/or operative report; iii) planning including typically radiography, computed tomography (CT), positron emission tomography (PET), or magnetic resonance imaging (MRI), as known by the skilled person. Medical imaging technologies using X-rays, such as CT scanner, are commonly used technologies to determine tumor bed treatment planning.

Computed tomography (CT) imaging is based on the variable absorption of X-rays by different tissues, and provides a cross-sectional imaging. The term "tomography" derives from the Greek term "tomos" meaning "slice" or "section" and "graphe" meaning "drawing". A CT imaging system produces cross-sectional images of the bones and soft tissues inside the body. CT images can be combined to create 3D images.

The nanoparticles and/or nanoparticles aggregates used in the context of the present description comprise or consist in an inorganic material preferably comprising at least one metal element with an atomic number of at least 25, preferably of at least 40, even more preferably above 40. The nanoparticles are intrinsically radio-opaque (i.e. they absorb X-rays) and can be easily visualized typically through radiography or computed tomography. When exposed to X-rays, typically delivered by CT scanner, the nanoparticles and/or nanoparticles aggregates create a marked contrast in the CT images due to the difference of electron density of the target biological tissues and of the particles.

The Hounsfield number is a normalized value of the calculated X-ray absorption coefficient of a pixel (picture element) in a computed tomogram. This number is expressed in Hounsfield units (HU). The CT number of air is -1000 (HU = -1000) and that of water is 0 (HU = 0). For inorganic particles with a high $Z_{eff}$, separation between tissues and particles occurs typically around HU values of 150. Above HU values of typically 120 up to 200, no more soft tissues densities can be measured.

The biocompatible gel comprising the nanoparticles and/or nanoparticles aggregates of the invention can be administered to the subject i) by deposition on the biological tissue of interest (targeted tissue) or ii) by filling the cavity left typically after a tumorectomy, preferably at the time of surgery (tumor resection).

Nanoparticles or aggregates of nanoparticles release from gel and then deposit on the targeted tissue, preferably on a tumor bed.

Preferably, the nanoparticles or aggregates of nanoparticles deposit on the targeted tissue typically between 24 hours and less than 1 month, preferably between 24 hours and 3 weeks, more preferably between 24 hours and 2 weeks, in order to allow for perfect and persistent targeted tissue delineation. Such delineation will be of high value, typically in the context of any further treatment planning. In a particular embodiment, the release and deposition of nanoparticles or aggregates of nanoparticles on the targeted tissue vary depending on gel viscosity (cf. examples 3, 4 and 7).

The biocompatible gel herein disclosed is for use for targeted tissue delineation.

When a cavity is to be filled with a gel according to the invention, the gel may fill at least 10% of the cavity's volume, preferably at least 20 % of the cavity's volume, even more preferably more than 30%, 40%, 50%, 60%, 70%, 80%, or 90% of the cavity's volume. 100% of the cavity's volume can also be filled with such a gel.

Repeated administrations of the gel can be performed, when appropriate.

The delineation of the targeted tissue allowed by the gel according to the present invention which comprises nanoparticles and/or aggregates of nanoparticles can be visualized using typically an X-ray medical imaging equipment, and more preferably a CT scanner. The term "delineate" means that the nanoparticles or aggregates of nanoparticles i) cover at least about 40%, preferably at least about 50%, and even more preferably more than about 50%, 60%, 70%, 80%, 90%, or about 95% of the targeted tissue; and preferably ii) form on the surface of the targeted tissues a layer with a thickness comprised between 100 µm and 0.5 cm, for example between 500 µm and 0.5 cm. The Hounsfield (HU) number within the layer is of at least 120 HU. Ideally, the nanoparticles or aggregates of nanoparticles cover 99% or even 100% of the targeted tissues.

Also herein described is a method of delineating a tumor bed in a subject, such a delineation allowing the subsequent visualization of said tumor bed using an X-ray imaging equipment, said method comprising exposing the tumor bed of a subject to a biocompatible gel comprising the nanoparticles or nanoparticles aggregates (as herein described), typically through deposition of the gel into the tumor bed, preferably at the time of surgery (tumor resection), in order to obtain the delineation of the tumor bed in a delay comprised between 24 hours and less than 1 month, preferably between 24 hours and 3 weeks, more preferably between 24 hours and 2 weeks following deposition. The tumor bed delineation can then be visualized by using X-ray imaging equipment.

The invention can be used to delineate any tumor bed of any type of malignant solid tumors, in particular of epithelial, neuroectodermal or mesenchymal origin, as well as lymphatic cancers so long as lymphatic nodes are concerned.

The biocompatible gel comprising the nanoparticles and/or aggregates of nanoparticles herein described is in particular intended to be used in the context of a cancer treatment protocol where radiotherapy is a classical adjuvant treatment or is the most appropriate adjuvant treatment for a particular subject, or where radiotherapy could be indicated as adjuvant treatment. Such cancer may be selected in particular from the group consisting of skin cancer, including malignant neoplasms associated to AIDS, melanoma; squamous cancer; central nervous system tumors including brain, cerebellum, pituitary, spinal cord, brainstem, eye and orbit; head and neck tumors; lung cancers; breast cancers; gastrointestinal tumors such as liver and hepatobiliary tract cancers, colon, rectum and anal cancers, stomach, pancreas, oesophagus cancer; male genitourinary tumors such as prostate, testis, penis and urethra cancers; gynecologic tumors such as uterine cervix, endometrium, ovary, fallopian tube, vagina and vulvar cancers; adrenal and retroperitoneal tumors; sarcomas of bone and soft tissue regardless the localization; and pediatric tumors such as Wilm's tumor, neuroblastoma, central nervous system tumors, Ewing's sarcoma, etc.

## BIOLOGICAL TISSUES AND TUMOR BED IRRADIATION

The biocompatible gel of the invention can be used in many fields, particularly in human or veterinary medicine. The biocompatible gel according to the invention, as herein described, is preferably for use in a mammal, even more preferably in a human being, as therapeutic agent in oncology, particularly when the nanoparticles or nanoparticles aggregates are exposed to ionizing radiations. Ionizing radiation is preferably selected from X-rays, gamma-rays and electron beam.

In a preferred embodiment, when the gel is applied on a target biological tissue, nanoparticles and/or nanoparticles aggregates of the biocompatible gel allow a at least about 10% increase of the radiation dose deposit on said target biological tissue when exposed to ionizing radiations, when compared to the dose deposit on the same biological tissue in the absence of said gel.

Typically, herein disclosed is a method of treatment allowing a radiation dose deposit enhancement of at least 10 % in a target tissue of a subject, preferably in a tumor bed of a subject, comprising the following steps in order of:

i) exposing the tumor bed of a subject to a biocompatible gel comprising the nanoparticles or nanoparticles aggregates according to the invention (as herein described), typically through deposition of the gel into the tumor bed, preferably at the time of surgery (tumor resection), in order to obtain the delineation of the tumor bed, and
ii) irradiating said nanoparticles or nanoparticles aggregates using ionizing radiation beam(s), thereby treating the subject.

Typically, the subject is a cancer patient.

Under the effect of ionizing radiations, in particular X-Rays, gamma-rays, radioactive isotopes and/or electron beams, the nanoparticles and/or nanoparticles aggregates generate electrons and/or high energy photons. Those electrons and/or high energy photons emitted after ionization will be responsible for direct and/or indirect cells damages, via free radicals generation, and ultimately for cells destruction, resulting in a better outcome for the patient.

The particles can be exposed to a large range of total dose of radiations.

Amounts and schedules (planning and delivery of irradiations in a single dose, or in the context of a fractioned or hyperfractioned protocol, etc.) is defined for any disease/anatomical site/disease stage patient setting/patient age (children, adult, elderly patient), and constitutes the standard of care for any specific situation.

As indicated previously, appropriate radiations or sources of excitation are preferably ionizing radiations and can advantageously be selected from the group consisting of X-Rays, gamma-Rays, electron beams, ion beams and radioactive isotopes or radioisotopes emissions. X-Rays and electrons beams are particularly preferred sources of excitation.

Ionizing radiations are typically of about 2 KeV to about 25 000 KeV (or 25 MeV), in particular of about 2 KeV to about 6000 KeV (i.e. 6 MeV) (LINAC source), or of about 50 KeV to about 25 000 KeV.

In general and in a non-restrictive manner, the following X-Rays can be applied in different cases to excite the particles:

- Superficial X-Rays of 2 to 50 keV : to excite nanoparticles near the surface (penetration of a few millimeters);
- X-Rays of 50 to 150 keV: in diagnostic but also in therapy;
- X-Rays (ortho voltage) of 200 to 500 keV which can penetrate a tissue thickness of 6 cm;
- X-Rays (mega voltage) of 1000 keV to 25,000 keV.

Radioactive isotopes can alternatively be used as an ionizing radiation source (named as curietherapy or brachytherapy). In particular, Iodine $I^{125}$ (t ½ =60.1 days), Palladium $Pd^{103}$ (t ½ = 17 days), Cesium $Cs^{137}$ and Iridium $Ir^{192}$ can advantageously be used.

Charged particles such as proton beams, ions beams such as carbon, in particular high energy ion beams, can also be used as a ionizing radiation source and/or neutron beams. Electron beams may also be used as a ionizing radiation source with energy comprised between 4 MeV and 25 MeV.

Specific monochromatic irradiation source could be used for selectively generating X-rays radiation at energy close to or corresponding to the desired X-ray absorption edge of the atoms ("metal element") constituting inorganic nanoparticles or nanoparticles aggregates. A preferred source of ionizing radiations is Linear Accelerator (LINAC).

A further object herein disclosed relates to a kit comprising a biocompatible gel comprising nanoparticles and/or nanoparticles aggregates (as herein described), optionally together with a therapeutic agent. In a particular embodiment, the kit comprises, in distinct containers, a biocompatible gel as herein described and a suspension of nanoparticles or nanoparticles aggregates as herein described (which are intended to be contacted, typically mixed, either *in situ,* i.e. on the target site, or *ex vivo* before deposition of the mixture on the target site).

A kit comprising a biocompatible gel comprising nanoparticles and/or nanoparticles aggregates as herein described, wherein the biocompatible gel and the nanoparticles and/or nanoparticles aggregates are in distinct containers is thus herein further described.

The following examples illustrate the invention without limiting its scope.

## BRIEF DESCRIPTION OF THE FIGURES

**FIGURE 1:** Tumor tissue delineation using clips

From *"Improving the definition of the tumor bed boost with the use of surgical clips and image registration in breast cancer patients"* [Int. J. Radiation Oncology Biol. Phys. Vol 78(5) ; 1352-1355 (2010)]. Tumor bed volume delineation: gross tumor volume (GTV) (red); clinical target volume (CTV) clips = all clips with 0.5-cm margins; planning target volume (PTV) (green) = GTV+CTV clips+0.5-cm lateral and 1-cm superior-inferior margins.

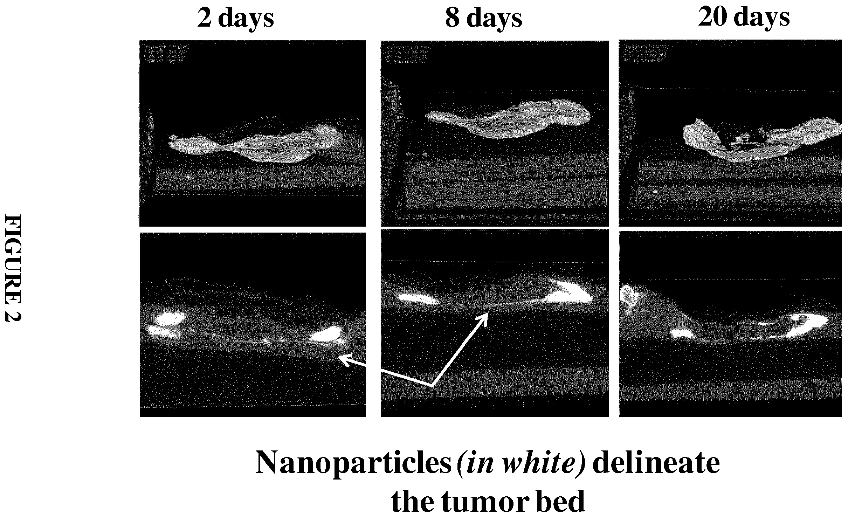

**FIGURE 2:** Micro-CT ($\mu$CT) images captured 2 days, 8 days and 20 days after gel deposition into the cavity obtained following tumorectomy, showing the tumor bed delineation using a biocompatible hydrogel according to the invention, composed of methylcellulose (5% w/w) comprising biocompatible nanoparticles and/or nanoparticles aggregates (3.5% w/w) consisting in hafnium oxide. The nanoparticles and/or nanoparticles aggregates have been mixed with the gel prior to the gel deposition into the tumor bed.

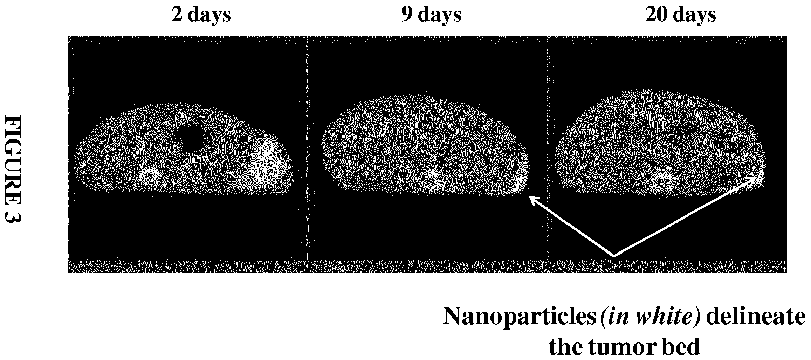

**FIGURE 3:** CT images captured 2 days, 9 days and 20 days after gel deposition into the cavity obtained following tumorectomy showing the tumor bed delineation using a biocompatible hydrogel according to the invention, composed of methylcellulose (9% w/w) comprising biocompatible nanoparticles and/or nanoparticles aggregates (3.5% w/w) consisting in hafnium oxide. The nanoparticles and/or nanoparticles aggregates have been mixed with the gel prior to the gel deposition into the tumor bed.

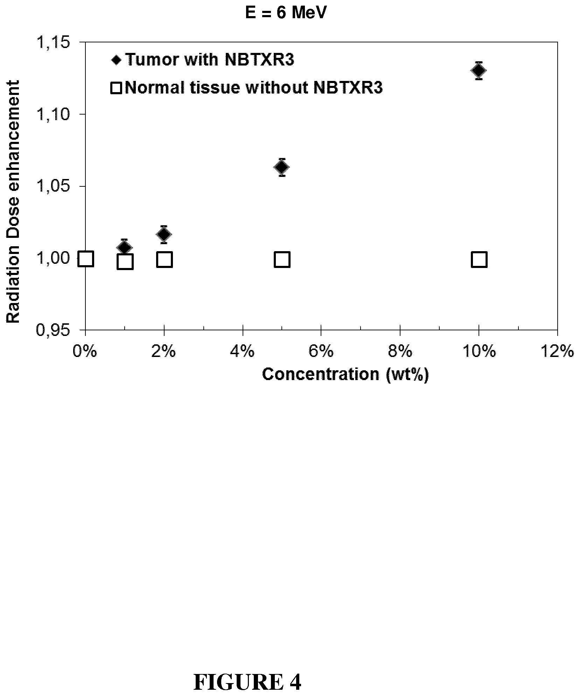

**FIGURE 4:** Effect of particles concentration on radiation dose enhancement using Monte Carlo calculation.

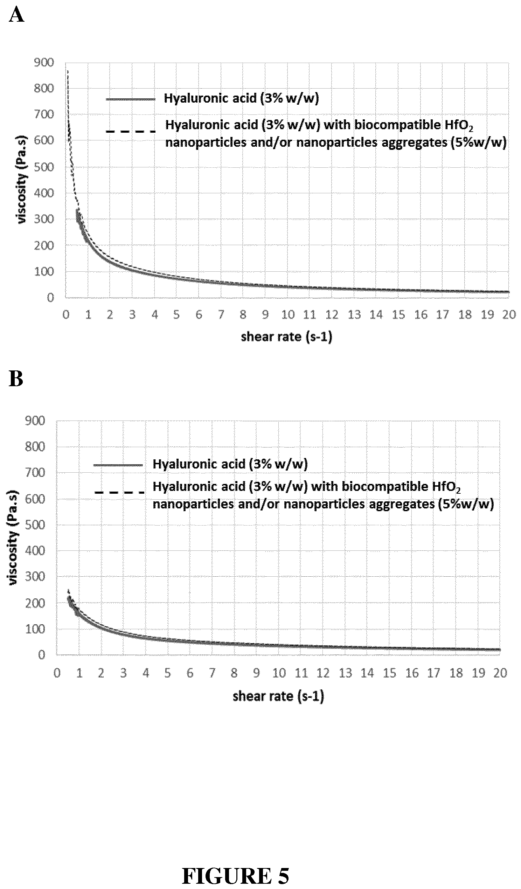

**FIGURE 5:** viscosity measurement (A at 20°C and B at 37°C) of a gel composed of hyaluronic acid (3% w/w) and a gel composed of hyaluronic acid (3% w/w) comprising biocompatible nanoparticles and/or nanoparticles aggregates (5% w/w) consisting in hafnium oxide.

**FIGURE 6:** FTIR spectrum of a gel composed of hyaluronic acid (0.1% w/w) comprising biocompatible nanoparticles and/or nanoparticles aggregates (0.26% w/w) consisting in hafnium oxide. Comparison with the spectrum of the gel composed of hyaluronic acid and also with the spectrum of nanoparticles and/or nanoparticles aggregates consisting in hafnium oxide.

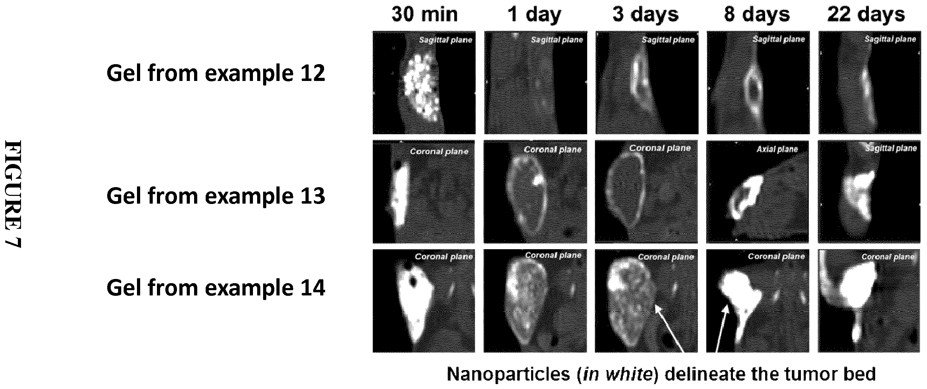

**FIGURE 7:** CT images captured 30 minutes (D01), 1 day (D02), 3 days (D04), 8 days (D09) and 22 days (D23) after gel deposition into the cavity obtained following tumorectomy showing the tumor bed delineation using a biocompatible hydrogel according to the invention. The gel is composed of hyaluronic acid (3.8% w/w), hyaluronic acid (2,5% w/w) and auto-cross-linked hyaluronic acid (3% w/w) comprising biocompatible nanoparticles and/or nanoparticles aggregates (5% w/w) consisting in hafnium oxide. The nanoparticles and/or nanoparticles aggregates have been mixed with the gel prior to the gel deposition into the tumor bed.

## EXAMPLES

### EXAMPLE 1: Biocompatible hafnium oxide ($HfO_2$) nanoparticles or nanoparticle aggregates, using sodium hexametaphosphate as coating agent.

A tetramethylammonium hydroxide (TMAOH) solution is added to HfCl4 solution. Addition of TMAOH solution is performed until the pH of the final suspension reaches a pH comprised between 7 and 13. A white precipitate is obtained.
The precipitate is further transferred in an autoclave and heated at a temperature comprised between 120 °C and 300 °C to perform crystallization. After cooling, the suspension is washed with de-ionized water.
Sodium hexametaphosphate solution is then added to the washed suspension and the pH is adjusted to a pH comprised between 6 and 8.
Sterilization of the nanoparticles or nanoparticles aggregates suspension is performed prior *in vitro* or *in vivo* experiments.

### EXAMPLE 2: Synthesis and physico-chemical characterisation of gold nanoparticles with different sizes.

Gold nanoparticles are obtained by reduction of gold chloride with sodium citrate in aqueous solution. Protocol was adapted from G. Frens Nature Physical Science 241 (1973) 21.
In a typical experiment, $HAuCl_4$ solution is heated to boiling. Subsequently, sodium citrate solution is added. The resulting solution is maintained under boiling for an additional period of 5 minutes.
The nanoparticle's size is adjusted from 15 nm up to 105 nm by carefully modifying the citrate versus gold precursor ratio (cf. Table 1).
The as prepared gold nanoparticles' suspensions are then concentrated using an ultrafiltration device with a 30 kDa cellulose membrane.
The resulting suspensions are ultimately filtered through a 0.22 $\mu$m cutoff membrane filter under laminar hood and stored at 4°C.
Particle size is determined on more than 200 particles, by using Transmission Electronic Microscopy (TEM) and by considering the longest nanoparticle dimension of each particle.

Table 1:

| Samples | Particle size (nm) | Synthesis Citrate | $HAuCl_4$ |
|---------|-------------------|-------------------|-----------|
| Gold-15 | 15±2 (1$\sigma$) | 20 mL 30 mL | 500 mL 0.25 mM |
| Gold-30 | 32±10 (1$\sigma$) | 7.5 mL 40 mM | 500 mL 0.25 mM |
| Gold-60 | 60±10 (1$\sigma$) | 2 mL 85 mM | 500 mL 0.25 mM |
| Gold-80 | 80± 10(1$\sigma$) | 1.2 mL 43 mM | 200 mL 0.30 mM |
| Gold-105 | 105±25 (1$\sigma$) | 1.2 mL 39 mM | 200 mL 0.33 mM |

### EXAMPLE 3: Biocompatible hafnium oxide nanoparticles and/or nanoparticles aggregates incorporation (3.5% w/w) within the gel (methylcellulose 5% w/w) prior gel deposition on the tumor bed.

A volume of aqueous suspension of biocompatible $HfO_2$ nanoparticles from example 1, is added to a volume of gel, typically with a polymer (methylcellulose) concentration lying between 4,5% w/w and 5,5 % w/w. The volumes' ratio between the suspension of $HfO_2$ nanoparticles and the gel being adjusted to reach a final $HfO_2$ nanoparticle concentration within gel of 3.5% (w/w). The so obtained preparation is mixed typically with a magnetic stirrer or a spatula.

**EXAMPLE 4: Biocompatible hafnium oxide Nanoparticles and/or nanoparticles aggregates incorporation (3.5% w/w) within the gel (methylcellulose 9% w/w) prior gel deposition on the tumor bed.**

A volume of aqueous suspension of biocompatible $HfO_2$ nanoparticles from example 1, is added to a volume of gel, typically with a polymer (methylcellulose) concentration lying between 8,5% w/w and 9,5 % w/w. The volumes' ratio between the suspension of $HfO_2$ nanoparticles and the gel being adjusted to reach a final $HfO_2$ nanoparticle concentration within gel of 3.5% (w/w).

**EXAMPLE 5: Assessment by micro-Computed Tomography ($\mu$CT) of the quality of the "tumor bed" delineation obtained when using nanoparticles embedded in hydrogel from example 3.**

The objective of this experiment was to assess, by $\mu$CT (Computed Tomography), the quality of "tumor bed" delineation by nanoparticles (NPs).
The test gel from example 3 was implanted (deposited) into the cavity left by the resection of HCT 116 xenografted tumor (human colorectal carcinoma cancer cells) in nude mice.
The $\mu$CT analysis was performed 2 days, 8 days and 20 days following gel implantation into the cavity left by the resection of the tumor in order to evaluate the volume occupied by the nanoparticles and/or nanoparticles aggregates in the tumor bed over time. For this, a manual segmentation (region of interest (ROIs)) was performed around the surgical cavity. Then, a thresholding above 120 HU was performed inside the surgical cavity in order to evaluate the presence of nanoparticles or nanoparticles aggregates and to assess both the location and volume occupied by those nanoparticles or nanoparticles aggregates for all mice. Figure 2 presents the $\mu$CT images showing more than about 80% of cavity delineation as soon as 2 days following surgery and gel implantation.

**EXAMPLE 6: Assessment by Computed Tomography (CT) of the quality of "tumor bed" delineation obtained when using nanoparticles embedded in hydrogel from example 4.**

The objective of this experiment was to assess, by CT (Computed Tomography), the quality of "tumor bed" delineation by nanoparticles (NPs).
The test gel from example 4 was implanted (deposited) into the cavity left by the resection of HCT 116 xenografted tumor (human colorectal carcinoma cancer cells) in nude mice.
The CT analysis was performed 2 days, 9 days and 20 days following gel implantation into the cavity left by the resection of the tumor in order to evaluate the volume occupied by the nanoparticles and/or nanoparticles aggregates in the tumor bed over time. For this, a manual segmentation (region of interest (ROIs)) was performed around the surgical cavity. Then, a thresholding above 120 HU was performed inside the surgical cavity in order to evaluate the presence of nanoparticles or nanoparticles aggregates and to assess both the location and volume occupied by those nanoparticles or nanoparticles aggregates for all mice. Figure 3 presents the CT images showing more than about 80% of cavity delineation as soon as 9 days following surgery and gel implantation.
Of note, gels comprising nanoparticles or nanoparticles aggregates prepared according to the protocols appearing in examples 3 and 4 have viscosity values at 2 s$^{-1}$ which are respectively equal to 190 Pa.s and 720 Pa.s at 37°C. Following their release from gels, the deposition of nanoparticles and/or nanoparticles aggregates on the tumor bed typically occurs within 2 days and 9 days respectively (see Figures 2 and 3).

**EXAMPLE 7: Calculation of the radiation dose deposit increase when nanoparticles and/or nanoparticles aggregates are present on the tumor bed from the estimation of nanoparticles or nanoparticles aggregates' mean concentration on the tumor bed.**

Table 2 presents calculated concentrations of any nanoparticles or nanoparticles aggregates as mentioned in claim 1 when the particles delineate the tumor bed; The initial concentrations of nanoparticles or nanoparticles aggregates within gel were chosen at 1% (w/w) and 3.5% (w/w). The tumor bed volume was calculated assuming different diameters of tumor bed, said diameters being between 1cm and 9 cm, while taking into account the diameter of the excised tumor as well as macroscopic margins. The thickness of the layer formed by deposition of the nanoparticles on the tumor bed was assumed to be respectively equal to 0.1, 0.5, 1 and 2 mm. The calculated nanoparticles or nanoparticles concentrations in those layers (nanoparticles concentration in the rim - cf . table 2) above 100g/l are underlined in bold characters. Figure 4 shows effect of particles concentration on radiation dose enhancement using Monte Carlo calculation.
Radiation dose enhancement was performed using a 'global model' calculation and a 6-MeV photon beam for both tumor with deep anatomical localization (with nanoparticles as mentioned in claim 1 composed of hafnium oxide herein identified as "NBTXR3 nanoparticles") and normal tissues (without nanoparticles). A $Z_{global}$ was used for the calculation.
In the global model calculation, the radiation dose enhancement (defined as the dose deposition in the tumor with high Z nanoparticles divided by dose deposition in the tumor without nanoparticles) results from energy deposition when considering an averaged Z value ($Z_{global}$) equal to

$$Z_{global} = (100\text{-}x) \times Z_{water} + x \times Z_{nanoparticles},$$

where "x" represents the concentration of nanoparticles within the tumor (mass of nanoparticles divided by the mass of the tumor), $Z_{water}$ represents the effective atomic number of water and $Z_{nanoparticles}$ the effective atomic number of the nanoparticles (i.e. hafnium oxide nanoparticles). In the calculation, the tumor was considered has having an effective atomic number equal to that of water. The nanoparticles increased the average efficacy of X-ray absorption in an isotropic fashion.

Results from figure 4 show that a 10% increase of radiation dose deposit is obtained for a nanoparticles concentration within tumor equal to or above 10% (wt%).

Based on results from figure 4 of "nanoscale Radiotherapy with Hafnium Oxide Nanoparticles" [Future Oncology 8(9),1167-1181 (2012)], and according to tables 2 A and 2B, a radiation dose deposit of at least 10% is obtained following nanoparticles and/or nanoparticles aggregates delineation of the tumor bed and the subsequent irradiation of said nanoparticles and/or nanoparticles aggregates, when using a biocompatible gel according to the invention, i.e., a biocompatible gel comprising nanoparticles and/or nanoparticles aggregates, wherein i) the density of each nanoparticle and nanoparticles aggregate is of at least 7 g/cm$^3$, the nanoparticle or nanoparticles aggregate comprising an inorganic material comprising at least one metal element having an atomic number Z of at least 25, preferably of at least 40, each of said nanoparticle and of said nanoparticles aggregate being covered with a biocompatible coating; ii) the nanoparticles and/or nanoparticles aggregates concentration is of at least about 1% (w/w); and iii) the apparent viscosity at 2 s$^{-1}$ of the gel comprising nanoparticles and/or nanoparticles aggregates is between about 0.1 Pa.s and about 1000 Pa.s when measured between 20°C and 37°C.

Table 2 A: concentration of nanoparticles in the rim assuming an initial nanoparticles concentration within gel of 10 g/L.

| Tumor diameter and margin (i.e. tumor excision including typically between 0,5 and 2 cm of macroscopic margin) (m) | Tumor bed radius (m) | Tumor bed volume (m$^3$) | Nanoparticles concentration within Gel (g/m$^3$) | Nanoparticles quantity within tumor bed (g) | Delineation of Nanoparticles following deposition on the tumor bed: Calculation of the volume of the Rim (m$^3$) | | | |
|---|---|---|---|---|---|---|---|---|
| | | | | | Rim = 0,1 mm | Rim = 0,5 mm | Rim = 1 mm | Rim = 2 mm |
| 0,010 | 0,005 | 5,24E-07 | 10000 | 0,0052 | 3,08E-08 | 1,42E-07 | 2,56E-07 | 4,11E-07 |
| 0,020 | 0,010 | 4,19E-06 | 10000 | 0,0419 | 1,24E-07 | 5,98E-07 | 1,14E-06 | 2,04E-06 |
| 0,030 | 0,015 | 1,41E-05 | 10000 | 0,1414 | 2,81E-07 | 1,37E-06 | 2,64E-06 | 4,94E-06 |
| 0,040 | 0,020 | 3,35E-05 | 10000 | 0,3351 | 5,00E-07 | 2,45E-06 | 4,78E-06 | 9,08E-06 |
| 0,050 | 0,025 | 6,55E-05 | 10000 | 0,6546 | 7,82E-07 | 3,85E-06 | 7,54E-06 | 1,45E-05 |
| 0,060 | 0,030 | 1,13E-04 | 10000 | 1,1311 | 1,13E-06 | 5,56E-06 | 1,09E-05 | 2,11E-05 |
| 0,070 | 0,035 | 1,80E-04 | 10000 | 1,7962 | 1,54E-06 | 7,59E-06 | 1,50E-05 | 2,91E-05 |
| 0,080 | 0,040 | 2,68E-04 | 10000 | 2,6812 | 2,01E-06 | 9,93E-06 | 1,96E-05 | 3,82E-05 |

(continued)

| Tumor diameter and margin (i.e. tumor excision including typically between 0,5 and 2 cm of macroscopic margin) (m) | Tumor bed radius (m) | Tumor bed volume (m$^3$) | Nanoparticles concentration within Gel (g/m$^3$) | Nanoparticles quantity within tumor bed (g) | Delineation of Nanoparticles following deposition on the tumor bed: Calculation of the volume of the Rim (m$^3$) | | | |
|---|---|---|---|---|---|---|---|---|
| | | | | | Rim = 0,1 mm | Rim = 0,5 mm | Rim = 1 mm | Rim = 2 mm |
| 0,090 | 0,045 | 3,82E-04 | 10000 | 3,8175 | 2,54E-06 | 1,26E-05 | 2,49E-05 | 4,87E-05 |

| Concentration of Nanoparticles in the Rim (g/l) | | | |
|---|---|---|---|
| Rim = 0,1 mm | Rim = 0,5 mm | Rim = 1 mm | Rim = 2 mm |
| 170 | 37 | 20 | 13 |
| 337 | 70 | 37 | 20 |
| 503 | 103 | 53 | 29 |
| 670 | 137 | 70 | 37 |
| 837 | 170 | 87 | 45 |
| 1003 | 203 | 103 | 53 |
| 1170 | 237 | 120 | 62 |
| 1337 | 270 | 137 | 70 |
| 1503 | 303 | 153 | 78 |

Table 2 B: concentration of nanoparticles in the rim assuming an initial nanoparticles concentration within gel of 35 g/L.

| Tumor diameter and margin (i.e. tumor excision including typically between 0,5 and 2 cm of macroscopic margin) (m) | Tumor bed radius (m) | Tumor bed volume (m$^3$) | Nanoparticles concentration within Gel (g/m$^3$) | Nanoparticles quantity within tumor bed (g) | Delineation of Nanoparticles following deposition on the tumor bed: Calculation of the volume of the Rim (m$^3$) | | | |
|---|---|---|---|---|---|---|---|---|
| | | | | | Rim = 0,1 mm | Rim = 0,5 mm | Rim = 1 mm | Rim = 2 mm |
| 0,01 | 0,005 | 5,24E-07 | 35000 | 0,0183 | 3,08E-08 | 1,42E-07 | 2,56E-07 | 4,11E-07 |
| 0,02 | 0,010 | 4,19E-06 | 35000 | 0,1466 | 1,24E-07 | 5,98E-07 | 1,14E-06 | 2,04E-06 |
| 0,03 | 0,015 | 1,41E-05 | 35000 | 0,4949 | 2,81E-07 | 1,37E-06 | 2,64E-06 | 4,94E-06 |
| 0,04 | 0,020 | 3,35E-05 | 35000 | 1,1730 | 5,00E-07 | 2,45E-06 | 4,78E-06 | 9,08E-06 |

(continued)

| Tumor diameter and margin (i.e. tumor excision including typically between 0,5 and 2 cm of macroscopic margin) (m) | Tumor bed radius (m) | Tumor bed volume (m³) | Nanoparticles concentration within Gel (g/m³) | Nanoparticles quantity within tumor bed (g) | Delineation of Nanoparticles following deposition on the tumor bed: Calculation of the volume of the Rim (m³) | | | |
|---|---|---|---|---|---|---|---|---|
| | | | | | Rim = 0,1 mm | Rim = 0,5 mm | Rim = 1 mm | Rim = 2 mm |
| 0,05 | 0,025 | 6,55E-05 | 35000 | 2,2910 | 7,82E-07 | 3,85E-06 | 7,54E-06 | 1,45E-05 |
| 0,06 | 0,030 | 1,13E-04 | 35000 | 3,9589 | 1,13E-06 | 5,56E-06 | 1,09E-05 | 2,11E-05 |
| 0,07 | 0,035 | 1,80E-04 | 35000 | 6,2866 | 1,54E-06 | 7,59E-06 | 1,50E-05 | 2,91E-05 |
| 0,08 | 0,040 | 2,68E-04 | 35000 | 9,3841 | 2,01E-06 | 9,93E-06 | 1,96E-05 | 3,82E-05 |
| 0,09 | 0,045 | 3,82E-04 | 35000 | 13,3614 | 2,54E-06 | 1,26E-05 | 2,49E-05 | 4,87E-05 |

| Concentration of Nanoparticles in the Rim (g/l) | | | |
|---|---|---|---|
| Rim = 0,1 mm | Rim = 0,5 mm | Rim = 1 mm | Rim = 2 mm |
| 595 | 129 | 72 | 45 |
| 1178 | 245 | 129 | 72 |
| 1762 | 362 | 187 | 100 |
| 2345 | 479 | 245 | 129 |
| 2928 | 595 | 304 | 158 |
| 3512 | 712 | 362 | 187 |
| 4095 | 828 | 420 | 216 |
| 4678 | 945 | 479 | 245 |
| 5262 | 1062 | 537 | 275 |

**EXAMPLE 8 : Biocompatible hafnium oxide nanoparticles and/or nanoparticles aggregates incorporation (5% w/w) within a hyaluronic acid gel (3% w/w).**

A volume of aqueous suspension of biocompatible $HfO_2$ nanoparticles from example 1, is added to a volume of gel, typically with a polymer (hyaluronic acid) concentration lying between 2.5% w/w and 4 % w/w. The volumes' ratio between the suspension of $HfO_2$ nanoparticles and the gel is adjusted to reach a final $HfO_2$ nanoparticle concentration within the gel of 5% (w/w). The so obtained preparation is mixed typically with a magnetic stirrer or a spatula.

**EXAMPLE 9 :viscosity measurement of a gel composed of hyaluronic acid (3%w/w) and a gel from example 8 composed of hyaluronic acid (3%w/w) comprising nanoparticles and/or nanoparticles aggregates (5% w/w) consisting in hafnium oxide.**

Viscosity measurement is typically performed, at 20°C and 37°C, using a Couette rheometer and following the standard DIN ISO 3219 recommendations (MODEL RM200, LAMY Rheology), on a given range of shear rate, lying between 0.1 s$^{-1}$ and 20 s$^{-1}$. The apparent viscosity is reported at 2s$^{-1}$. The absence of strong interaction between the particle and the polymer which forms the biocompatible gel can typically be verified by measuring the viscosity of the gel comprising the nanoparticles and/or nanoparticles aggregates at 20°C and 37°C, as described above, and by comparing the obtained viscosity curve with that of a gel comprising neither nanoparticles nor nanoparticles aggregates. The apparent viscosity at 2s$^{-1}$ for both gels is higher than 150 Pa.s at 20 °C and higher than 100 Pa.s at 37 °C. The Similar viscosity curves observed (i.e. values differing one from each other by no more than 20%, typically by no more than 15%) confirm the absence of strong interaction between nanoparticles and/or nanoparticles aggregates and gel (cf. figures 5A and 5B).

**EXAMPLE 10: Biocompatible hafnium oxide nanoparticles and/or nanoparticles aggregates incorporation (0.26% w/w) within the gel of hyaluronic acid (0.1% w/w).**

A volume of aqueous suspension of biocompatible $HfO_2$ nanoparticles from example 1 is added to a volume of gel typically with a polymer (hyaluronic acid) concentration lying between 0.05% w/w and 0.25 % w/w. The volumes' ratio between the suspension of $HfO_2$ nanoparticles and the gel is adjusted to reach a final $HfO_2$ nanoparticle concentration within the gel of 0.26% (w/w). The so obtained preparation is mixed typically with a magnetic stirrer or a spatula.

**EXAMPLE 11: FTIR (Fourier Transform Infrared Spectroscopy) spectra of the gel of example 10 and comparison with a gel composed of hyaluronic acid and also with nanoparticles and/or nanoparticles aggregates.**

The bands observed in the gel embedding biocompatible hafnium oxide nanoparticles and/or nanoparticles aggregates correspond to the bands characteristic of the gel composed of hyaluronic acid and to the bands of nanoparticles and/or nanoparticles aggregates consisting in hafnium oxide. No characteristic bands of one or the other of the components are missing and no new bands appear. FTIR spectra show no signature revealing an interaction between nanoparticles and/or nanoparticles aggregates and gel (cf. Figure 6 and the below tables 3 and 4).

Table 3: FTIR bands assignment for hyaluronic acid (from Pasqui D. *et al.* Polysaccharide-based hydrogels: the key role of water in affecting mechanical properties. Polymers, Vol 4, p. 1517-1534, 2012).

| hyaluronic acid | |
|---|---|
| wavenumber (cm$^{-1}$) | assignment |
| 3310 | water molecules - OH |
| 2930 | -C-C-C-H stretching |
| 2875 | -C-C-C-H stretching |
| 1640 | amide -C=O stretching |
| 1610 | carboxylate asymm. Stretching |
| 1560 | amide N-H bending |
| 1410 | carboxylate asymm. Stretching |
| 1375 | C-CH and O-CH stretching |
| 1281 | -C-O stretching |
| 1146 | C-C C-O stretching |
| 1046 | C-O-C bending |

Table 4: FTIR bands assignment for hafnium oxide (from Ramadoss A. *et al.* Synthesis and characterization of $HfO_2$ nanoparticles by sonochemical approach. Journal of Alloys and Compounds, Vol 544, p. 115-119, 2012)

| $HfO_2$ | |
|---|---|
| wavenumber (cm$^{-1}$) | assignment |
| 3417 | stretching O-H |
| 1628 | bending H-O-H |
| 1011 | coating |

(continued)

| HfO$_2$ | |
|---|---|
| 755 | m-HfO$_2$ |
| 675 | m-HfO$_2$ |
| 523 | m-HfO$_2$ |
| 419 | m-HfO$_2$ |

**EXAMPLE 12: Biocompatible hafnium oxide Nanoparticles and/or nanoparticles aggregates' incorporation (5% w/w) within the gel (hyaluronic acid 3.8 % w/w) prior gel deposition on the tumor bed.**

A volume of aqueous suspension of biocompatible HfO$_2$ nanoparticles from example 1, is added to a volume of gel, typically with a polymer (hyaluronic acid) concentration lying between 3,3% w/w and 4,3 % w/w. The volumes' ratio between the suspension of HfO$_2$ nanoparticles and the gel being adjusted to reach a final HfO$_2$ nanoparticle concentration within gel of 5% (w/w).

**EXAMPLE 13: Biocompatible hafnium oxide Nanoparticles and/or nanoparticles aggregates' incorporation (5% w/w) within the gel (hyaluronic acid 2.5 % w/w) prior gel deposition on the tumor bed.**

A volume of aqueous suspension of biocompatible HfO$_2$ nanoparticles from example 1, is added to a volume of gel, typically with a polymer (hyaluronic acid) concentration lying between 2% w/w and 3% w/w. The volumes' ratio between the suspension of HfO$_2$ nanoparticles and the gel being adjusted to reach a final HfO$_2$ nanoparticle concentration within gel of 5% (w/w).

**EXAMPLE 14: Biocompatible hafnium oxide Nanoparticles and/or nanoparticles aggregates' incorporation (5% w/w) within the gel (auto-cross-linked hyaluronic acid 3 % w/w) prior gel deposition on the tumor bed.**

A volume of aqueous suspension of biocompatible HfO$_2$ nanoparticles from example 1, is added to a volume of gel, typically with a polymer (auto-cross-linked hyaluronic acid) concentration lying between 2.5% w/w and 3.5% w/w. The volumes' ratio between the suspension of HfO$_2$ nanoparticles and the gel being adjusted to reach a final HfO$_2$ nanoparticle concentration within gel of 5% (w/w).

**EXAMPLE 15: Assessment by Computed Tomography (CT) of the quality of "tumor bed" delineation obtained when using nanoparticles respectively embedded in hydrogels from examples 12, 13 and 14.**

The objective of this experiment was to assess, by CT (Computed Tomography), the quality of "tumor bed" delineation by nanoparticles (NPs). The test gels from examples 12 (Figure 7, top panel), example 13 (Figure 7, middle panel) and example 14 (Figure 7, bottom panel) were implanted (deposited) into the cavity left by the resection of EMT-6 orthotopic grafted tumor (murine breast carcinoma cancer cells) in BALB/cJRj mice. The CT analysis was performed 30 minutes, 1 day, 2 days, 8 days and 22 days following gel implantation into the cavity left by the resection of the tumor in order to evaluate the volume occupied by the nanoparticles and/or nanoparticles aggregates in the tumor bed over time. For this, a manual segmentation (region of interest (ROIs)) was performed around the surgical cavity. Then, a thresholding above 120 HU was performed inside the surgical cavity in order to evaluate the presence of nanoparticles or nanoparticles aggregates and to assess both the location and volume occupied by those nanoparticles or nanoparticles aggregates for all mice. Figure 7 presents the CT images showing more than about 80% of cavity delineation as soon as 3 days following surgery and gel implantation. Following their release from gels, the deposition of nanoparticles and/or nanoparticles aggregates on the tumor bed typically occurs within 3 days (see Figure 7).

REFERENCES

- Customized Computed Tomography-Based Boost Volumes in Breast-Conserving Therapy: Use of Three-Dimensional Histologic Information for Clinical Target Volume Margins. IJROB 75(3) 757-763 (2009)
- Target volume definition for external beam partial breast radiotherapy: clinical, pathological and technical studies informing current approaches. Radiotherapy and Oncology 94 255-263 (2010)

**EP 3 010 552 B1**

- Excised and Irradiated Volumes in Relation to the Tumor size in Breast-Conserving Therapy. Breast Cancer Res Treat 129:857-865 (2011)
- Guidelines for target volume definition in post-operative radiotherapy for prostate cancer, on behalf of the EORTC Radiation Oncology Group. Radiotherapy & Oncology 84 121-127 (2007)
- Improving the definition of the tumor bed boost with the use of surgical clips and image registration in breast cancer patients. Int. J. Radiation Oncology Biol. Phys. Vol 78(5) ; 1352-1355 (2010)
- The dynamic tumor bed: volumetric changes in the lumpectomy cavity during breast conserving therapy. Int. J. Radiation Oncology Biol. Phys. 74(3):695-701 (2009).
- Nanoscale Radiotherapy with Hafnium Oxide Nanoparticles. Future Oncology 8(9):1167-1181 (2012).
- Polysaccharide-based hydrogels: the key role of water in affecting mechanical properties. Polymers. 4: 1517-1534, 2(2012).
- Ramadoss A. et al. Synthesis and characterization of HfO2 nanoparticles by sonochemical approach. Journal of Alloys and Compounds. 544:115-119 (2012).

**Claims**

1. A biocompatible gel comprising nanoparticles and/or nanoparticles aggregates for use for treating cancer in a subject, wherein i) the density of each nanoparticle and nanoparticles aggregate is of at least 7 $g/cm^3$, the nanoparticle or nanoparticles aggregate consisting in an inorganic material comprising a single metal element or a mixture of metal elements, each metal element having an atomic number Z of at least 40, each of said nanoparticle and of said nanoparticles aggregate being covered with a biocompatible coating; ii) the nanoparticles and/or nanoparticles aggregates concentration is of at least about 1% (w/w); and iii) the apparent viscosity at $2s^{-1}$ of the gel comprising nanoparticles and/or nanoparticles aggregates is between about 0.1 Pa.s and about 1000 Pa.s when measured between 20°C and 37°C, and wherein the treatment of cancer comprises a step of exposing a target biological tissue of the subject to the gel wherein said gel delineates the target biological tissue and a step of irradiating the gel-delineated target biological tissue.

2. The biocompatible gel for use according to claim 1, wherein the nanoparticles and/or nanoparticles aggregates concentration is between about 1.5% and 10% (w/w).

3. The biocompatible gel for use according to claim 1 or 2, wherein the inorganic material is a metal, an oxide, a sulfide, or any mixture thereof.

4. The biocompatible gel for use according to anyone of claims 1 to 3, wherein the nanoparticle or nanoparticles aggregate further comprises at least one targeting agent.

5. The biocompatible gel for use according to anyone of claims 1 to 4, wherein the gel is an hydrogel.

6. The biocompatible gel for use according to anyone of claims 1 to 5, wherein, when the gel is applied on a target biological tissue, nanoparticles and/or nanoparticles aggregates of the biocompatible gel allow a at least about 10% increase of the radiation dose deposit on said target biological tissue when exposed to ionizing radiations, when compared to the dose deposit on the same biological tissue in the absence of said gel.

7. The biocompatible gel for use according to claim 6, wherein the applied ionizing radiation dose is between 2 KeV and 25 MeV.

8. The biocompatible gel for use according to claim 7, wherein the ionizing radiation is selected from X-rays, gamma rays and electron beam.

9. The biocompatible gel for use according to anyone of claims 6 to 8, wherein the gel allows the delineation and visualization of at least 40% of the target biological tissue.

10. The biocompatible gel for use according to anyone of claims 1 to 9, wherein the biological tissue is a tumor bed.

11. The biocompatible gel for use according to claim 10, wherein the tumor bed is the tissue covering the cavity obtained following tumor resection.

18

**12.** A kit comprising a biocompatible gel comprising nanoparticles and/or nanoparticles aggregates as described in anyone of claims 1 to 11, wherein the biocompatible gel and the nanoparticles and/or nanoparticles aggregates are in distinct containers.

**Patentansprüche**

**1.** Ein biokompatibles Gel umfassend Nanopartikel und/oder Nanopartikelaggregate zur Verwendung zur Behandlung von Krebs in einem Individuum, wobei i) die Dichte jedes Nanopartikels und Nanopartikelaggregats mindestens 7 g/cm$^3$ beträgt, das Nanopartikel oder Nanopartikelaggregat aus einem anorganischen Material besteht, das ein einzelnes Metallelement oder ein Gemisch von Metallelementen umfasst, wobei jedes Metallelement eine Ordnungszahl Z von mindestens 40 aufweist, und jedes der Nanopartikel und der Nanopartikelaggregate mit einer biokompatiblen Beschichtung umhüllt ist; ii) die Konzentration der Nanopartikel und/oder Nanopartikelaggregate mindestens etwa 1% (w/w) beträgt; und iii) die scheinbare Viskosität des Nanopartikel und/oder Nanopartikelaggregate umfassenden Gels bei 2s$^{-1}$ zwischen etwa 0.1 Pa.s und etwa 1000 Pa.s liegt, wenn zwischen 20°C und 37°C gemessen wird, und wobei die Krebsbehandlung einen Schritt der Exposition eines biologischen Zielgewebes des Individuums gegenüber dem Gel umfasst, wobei das Gel das biologische Zielgewebe abgrenzt, und einen Schritt des Bestrahlens des mit dem Gel abgegrenzten biologischen Zielgewebes umfasst.

**2.** Das biokompatible Gel zur Verwendung nach Anspruch 1, wobei die Konzentration der Nanopartikel und/oder Nanopartikelaggregate zwischen etwa 1,5% und 10% (w/w) liegt.

**3.** Das biokompatible Gel zur Verwendung nach Anspruch 1 oder 2, wobei das anorganische Material ein Metall, ein Oxid, ein Sulfid, oder eine beliebige Mischung davon ist.

**4.** Das biokompatible Gel zur Verwendung nach einem der Ansprüche 1 bis 3, wobei das Nanopartikel oder Nanopartikelaggregat ferner mindestens ein Targetting-Mittel umfasst.

**5.** Das biokompatible Gel zur Verwendung nach einem der Ansprüche 1 bis 4, wobei das Gel ein Hydrogel ist.

**6.** Das biokompatible Gel zur Verwendung nach einem der Ansprüche 1 bis 5, wobei, wenn das Gel auf das biologische Zielgewebe aufgebracht wird, die Nanopartikel und/oder Nanopartikelaggregate des biokompatiblen Gels mindestens einen etwa 10% Zuwachs der an das biologische Zielgewebe abgegebenen Strahlendosis ermöglichen, wenn dieses ionisierender Strahlung ausgesetzt wird, im Vergleich zu der abgegebenen Dosis auf das gleiche biologische Gewebe in Abwesenheit des Gels.

**7.** Das biokompatible Gel zur Verwendung nach Anspruch 6, wobei die angewendete Ionisierungsstrahlungsdosis zwischen 2 KeV und 25 MeV liegt.

**8.** Das biokompatible Gel zur Verwendung nach Anspruch 7, wobei die ionisierende Strahlung aus Röntgenstrahlung, Gammastrahlung und Elektronenstrahl ausgewählt ist.

**9.** Das biokompatible Gel zur Verwendung nach einem der Ansprüche 6 bis 8, wobei das Gel die Abgrenzung und Visualisierung von mindestens 40% des biologischen Zielgewebes ermöglicht.

**10.** Das biokompatible Gel zur Verwendung nach einem der Ansprüche 1 bis 9, wobei das biologische Gewebe ein Tumorbett ist.

**11.** Das biokompatible Gel zur Verwendung nach Anspruch 10, wobei das Tumorbett dasjenige Gewebe ist, das die nach einer Tumorresektion entstehende Kavität auskleidet.

**12.** Ein Kit umfassend ein biokompatibles Gel umfassend Nanopartikel und/oder Nanopartikelaggregate wie in einem der Ansprüche 1 bis 11 beschrieben, wobei sich das biokompatible Gel und die Nanopartikel und/oder Nanopartikelaggregate in eigenen Behältern befinden.

**Revendications**

1. Gel biocompatible comprenant des nanoparticules et/ou agrégats de nanoparticules pour une utilisation pour traiter le cancer chez un sujet, dans lequel i) la densité de chaque nanoparticule et agrégat de nanoparticules est d'au moins 7 g/cm$^3$, la nanoparticule ou l'agrégat de nanoparticules consistant en un matériel inorganique comprenant un élément métallique unique ou un mélange d'éléments métalliques, chaque élément métallique ayant un numéro atomique Z d'au moins 40, chaque dite nanoparticule et dit agrégat de nanoparticules étant couvert d'un revêtement biocompatible ; ii) la concentration des nanoparticules et/ou agrégats de nanoparticules est d'au moins environ 1% (p/p) ; et iii) la viscosité apparente à 2s$^{-1}$ du gel comprenant nanoparticules et/ou agrégats de nanoparticules est comprise entre environ 0.1 Pa.s et environ 1000 Pa.s lorsqu'elle est mesurée entre 20°C et 37°C, et dans lequel le traitement du cancer comprend une étape d'exposition d'un tissu biologique cible du sujet au gel dans laquelle ledit gel permet la délimitation du tissu biologique cible et une étape d'irradiation du tissu biologique cible délimité par le gel.

2. Gel biocompatible pour utilisation selon la revendication 1, dans lequel la concentration de nanoparticules et/ou agrégats de nanoparticules est comprise entre environ 1.5% et 10% (p/p).

3. Gel biocompatible pour utilisation selon la revendication 1 ou 2, dans lequel le matériel inorganique est un métal, un oxide, un sulfide, ou tout mélange de ceux-ci.

4. Gel biocompatible pour utilisation selon l'une quelconque des revendications 1 à 3, dans lequel la nanoparticule ou l'agrégat de nanoparticules comprend en outre au moins un agent de ciblage.

5. Gel biocompatible pour utilisation selon l'une quelconque des revendications 1 à 4, dans lequel le gel est un hydrogel.

6. Gel biocompatible pour utilisation selon l'une quelconque des revendications 1 à 5, dans lequel, lorsque le gel est appliqué sur un tissu biologique cible, les nanoparticules et/ou agrégats de nanoparticules du gel biocompatible permettent une augmentation de au moins environ 10% du dépôt de dose de rayonnement sur ledit tissu biologique cible sous exposition à des radiations ionisantes, par comparaison avec la dose déposée sur le même tissu biologique en l'absence dudit gel.

7. Gel biocompatible pour utilisation selon la revendication 6, dans lequel la dose de radiations ionisantes appliquée est comprise entre 2 KeV et 25 MeV.

8. Gel biocompatible pour utilisation selon la revendication 7, dans lequel la radiation ionisante est sélectionnée parmi rayons X, rayons gamma et faisceau d'électrons.

9. Gel biocompatible pour utilisation selon l'une quelconque des revendications 6 à 8, dans lequel le gel permet la délimitation et la visualisation d'au moins 40% du tissu biologique cible.

10. Gel biocompatible pour utilisation selon l'une quelconque des revendications 1 à 9, dans lequel le tissu biologique est un lit tumoral.

11. Gel biocompatible pour utilisation selon la revendication 10, dans lequel le lit tumoral est le tissu couvrant la cavité obtenue après résection tumorale.

12. Kit comprenant un gel biocompatible comprenant nanoparticules et/ou agrégats de nanoparticules tels que décrits dans l'une quelconque des revendications 1 à 11, dans lequel le gel biocompatible et les nanoparticules et/ou agrégats de nanoparticules se trouvent dans des contenants distincts.

**FIGURE 1**

**2 days**  **8 days**  **20 days**

FIGURE 2

Nanoparticles *(in white)* delineate
the tumor bed

FIGURE 3

FIGURE 4

**A**

**B**

# FIGURE 5

**FIGURE 7**

## REFERENCES CITED IN THE DESCRIPTION

### Patent documents cited in the description

- WO 2011084465 A

### Non-patent literature cited in the description

- Customized computed tomography-based boost volumes in breast-conserving therapy: use of three-dimensional histologic information for clinical target volume margins. *IJROBP,* 2009, vol. 75 (3), 757-763
- Target volume definition for external beam partial breast radiotherapy: clinical, pathological and technical studies informing current approaches. *Radiotherapy and Oncology,* 2010, vol. 94, 255-263
- Excised and Irradiated Volumes in Relation to the Tumor size in Breast-Conserving Therapy. *Breast Cancer Res Treat,* 2011, vol. 129, 857-865
- Guidelines for target volume definition in post-operative radiotherapy for prostate cancer, on behalf of the EORTC Radiation Oncology Group. *Radiotherapy & Oncology,* 2007, vol. 84, 121-127
- Improving the definition of the tumor bed boost with the use of surgical clips and image registration in breast cancer patients. *Int. J. Radiation Oncology Biol. Phys.,* 2010, vol. 78 (5), 1352-1355
- The dynamic tumor bed: volumetric changes in the lumpectomy cavity during breast conserving therapy. *Int. J. Radiation Oncology Biol. Phys.,* 2009, vol. 74 (3), 695-701
- **G. FRENS.** *Nature Physical Science,* 1973, vol. 241, 21
- nanoscale Radiotherapy with Hafnium Oxide Nanoparticles. *Future Oncology,* 2012, vol. 8 (9), 1167-1181
- Customized Computed Tomography-Based Boost Volumes in Breast-Conserving Therapy: Use of Three-Dimensional Histologic Information for Clinical Target Volume Margins. *IJROB,* 2009, vol. 75 (3), 757-763
- Nanoscale Radiotherapy with Hafnium Oxide Nanoparticles. *Future Oncology,* 2012, vol. 8 (9), 1167-1181
- Polysaccharide-based hydrogels: the key role of water in affecting mechanical properties. *Polymers,* 2012, vol. 4 (2), 1517-1534
- **RAMADOSS A. et al.** Synthesis and characterization of HfO2 nanoparticles by sonochemical approach. *Journal of Alloys and Compounds,* 2012, vol. 544, 115-119